(19) Europäisches Patentamt
European Patent Office
Office européen des brevets

(11) EP 0 680 972 B1

(12) **FASCICULE DE BREVET EUROPEEN**

(45) Date de publication et mention de la délivrance du brevet:
**08.03.2000 Bulletin 2000/10**

(51) Int Cl.[7]: **C07K 5/062**, C07K 5/065, A61K 38/05

(21) Numéro de dépôt: **95401043.5**

(22) Date de dépôt: **05.05.1995**

(54) **Peptides dérivés de trifluorométhylcétones, leur procédé de préparation et les compositions pharmaceutiques qui les contiennent**

Trifluormethylketonderivate von Peptiden, Verfahren zu ihrer Herstellung und sie enthaltende pharmazeutische Zusammensetzungen

Trifluoromethylketone peptide derivatives, process for their preparation and pharmaceutical compositions comprising them

(84) Etats contractants désignés:
**AT BE CH DE DK ES FR GB GR IE IT LI LU NL PT SE**

(30) Priorité: **05.05.1994 FR 9405494**

(43) Date de publication de la demande:
**08.11.1995 Bulletin 1995/45**

(73) Titulaire: **ADIR ET COMPAGNIE**
**92415 Courbevoie Cédex (FR)**

(72) Inventeurs:
- **de Nanteuil , Guillaume**
  **F-92150 Suresnes (FR)**
- **Portevin, Bernard**
  **F-78990 Elancourt (FR)**
- **Canet, Emmanuel**
  **F-75005 Paris (FR)**

(56) Documents cités:
**EP-A- 0 189 305** **EP-A- 0 585 155**

- **JOURNAL OF MEDICINAL CHEMISTRY, vol. 35, no. 4, 21 Février 1992 WASHINGTON US, pages 641-662, J.W. SKILES ET AL. 'Inhibition of Human Leukocyte Elastase (HLE) by N-Substituted Peptidyl Trifluoromethyl Ketones'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux peptides dérivés de trifluorométhylcétones, leur procédé de préparation et les compositions pharmaceutiques qui les contiennent.

Ces nouveaux dérivés peptidiques possèdent des propriétés inhibitrices de l'élastase leucocytaire humaine. De plus, ces composés possèdent des propriétés antioxydantes.

L'élastine est une protéine fibreuse élastique du tissu conjonctif des vertébrés. Elle est présente dans les parois vasculaires, la peau, les poumons, les cartilages, les ligaments et d'autres tissus. Les élastases sont des enzymes capables de solubiliser l'élastine fibreuse. L'élastase leucocytaire humaine est une sérine protéase, qui se trouve sous forme active dans les granules azurophiles du neutrophile polymorphonucléaire. C'est une glycoprotéine de 25 à 30 kDa formée de 218 acides aminés. L'élastase leucocytaire humaine (ELH) solubilise l'élastine fibreuse, mais clive également d'autres protéines de la matrice extracellulaire (collagènes, fibronectine, protéoglycanes, ...), hydrolyse et inactive un certain nombre de protéines plasmatiques (facteur de la coagulation, immunoglobuline, complément, ...). L'activité élastolytique est contrôlée et régulée par des inhibiteurs naturels (alpha 1 - antitrypsine, alpha 2 - macroglobuline, l'inhibiteur bronchique) sensibles aux oxydants.

Des inhibiteurs de l'élastase leucocytaire humaine, réversibles ou irréversibles ont été décrits dans la littérature pour le traitement de situations physiopathologiques où son rôle a été évoqué (D.A. TRAINOR, TIPS, 8, 303-307, 1987).

Ces états pathologiques peuvent être l'emphysème pulmonaire, l'arthrite rhumatoïde, les maladies dégénératives du tissu conjonctif comme l'athérosclérose (J.G. BIETH, "Elastases : Catalytic and Biological Properties" dans "Regulation of matrix accumulation" - R.P. MECHAM - Academic Press, NY, 217-320, 1986), le syndrome de détresse respiratoire aigue de l'adulte (P.M. SUTER et coll., Am. Rev. Respir. Dis., 145, 1016-1022, 1992), la fibrose kystique (K.C. MEYER et coll., Am. Rev. Respir. Dis., 144, 580-585, 1991), la bronchite chronique (J.A. NADEL, Respiration, 58 (suppl.1, 3-5), 1991), les glomérulonéphrites (E. SANDERS et coll., Renal. physiol., 3, 355-359, 1980), le psoriasis (J. SCHALKWIJK et coll., Br. J. Dermatology, 122, 631-644, 1990), les lésions tissulaires survenant au cours des processus d'ischémie-reperfusion (F.A. NICOLINI et coll., Am. HEART J., 122-1245, 1991 et C.R.B. WELBOURN et coll., Am. J. Physiol. 260, 1852-1856, 1991). Elle pourrait également jouer un rôle dans les phénomènes de migration cellulaire normale ou pathologique-invasion tumorale (J.G. BIETH cité plus haut).

Récemment des peptides dérivés de trifluorométhylcétones ont été décrits comme inhibiteurs d'ELH. C'est le cas plus particulièrement des composés décrits dans les brevets EP 189 305 EP 369 391 ou EP 585 155.

La présente invention concerne plus spécifiquement les composés de formule (I):

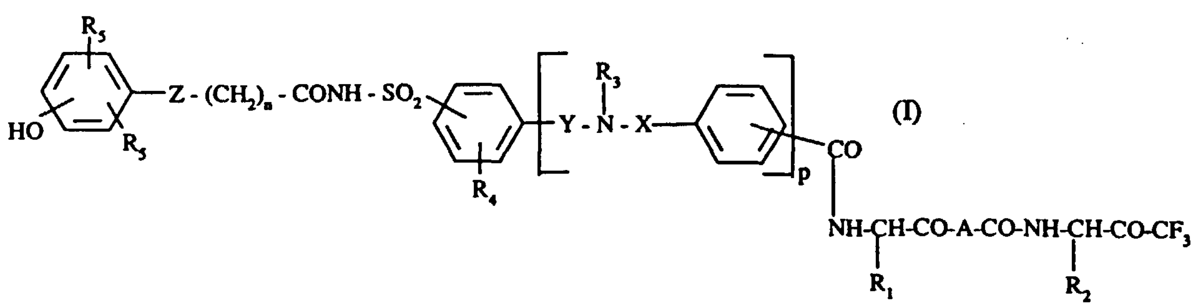

(I)

dans laquelle :

$R_1$ représente un groupement alkyle ($C_1$-$C_6$) linéaire ou ramifié ou cycloalkyle ($C_3$-$C_7$),

$R_2$ représente un groupement alkyle ($C_1$-$C_6$) linéaire ou ramifié ou cycloalkyle ($C_3$-$C_7$),

$R_3$ représente un atome d'hydrogène ou un groupement alkyle ($C_1$-$C_6$) linéaire ou ramifié,

$R_4$ représente un atome d'halogène ou un groupement alkyle ($C_1$-$C_6$) linéaire ou ramifié ou alkoxy ($C_1$-$C_6$) linéaire ou ramifié,

$R_5$ représente un groupement alkyle ($C_1$-$C_6$) linéaire ou ramifié,

X, Y, différents représentent CO ou $SO_2$,

n représente 1, 2 ou 3,
p représente 1,
Z représente un atome de soufre ou d'oxygène,

A représente l'un quelconque des groupements suivants :

- 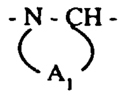

  dans lequel :

  $A_1$ représente avec les atomes d'azote et de carbone auquel il est attaché un cycle 2-azabicyclo[2.2.2] octane, 2-azabicyclo[2,2,1]heptane, perhydroindole, perhydroisoindole, indoline, isoindoline, perhydroquinoléine, perhydroisoquinoléine, 1,2,3,4-tétrahydroisoquinoléine, 1,2,3,4-tétrahydroquinoléine, cyclopenta[b]pyrolidine, 1,3-thiazolidine ou pyrrolidine,

- ou,

  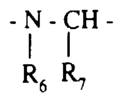 ,

  dans lequel :

  $R_6$ représente un atome d'hydrogène, un groupement alkyle (C1-C6) linéaire ou ramifié, un groupement cycloalkyle (C3-C6) ou un groupement indan-2-yle,
  $R_7$ représente un atome d'hydrogène ou un groupement alkyle (C1-C6) linéaire ou ramifié,

composés de formule (I) qui comprennent les hydrates de la fonction cétone $COCF_3$ correspondants,
leurs énantiomères, diastéréoisomères et épimères ainsi que leurs sels d'addition à une base pharmaceutiquement acceptable.
Parmi les bases pharmaceutiquement acceptables, on peut citer à titre non limitatif l'hydroxyde de sodium, l'hydroxyde de potassium, la tertbutylamine, la diéthylamine, l'éthylènediamine, etc...
L'invention s'étend également au procédé de préparation des composés de formule (I) caractérisé en ce que l'on utilise, comme produit de départ un alcool de formule (II), dont on a éventuellement séparé les isomères par une technique classique de séparation :

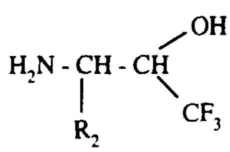 (II)

dans laquelle $R_2$ a la même signification que dans la formule (I),
que l'on fait réagir:

a soit sur un amino-acide protégé de formule (III), dont on a éventuellement séparé les isomères selon une technique classique de séparation, par une technique classique de couplage peptidique comme celle décrite par W. KONIG et R. GEIGER (Ber., 103, 788, 1970) :

$$Boc\text{-}A\text{-}CO_2H \qquad (III)$$

dans laquelle A a la même signification que dans la formule (I) et Boc représente un groupement tertbutoxycarbonyle,
pour conduire au composé de formule (IV) :

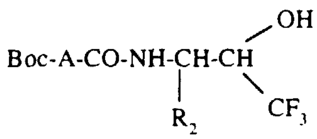 (IV)

dans laquelle A, $R_2$ et Boc ont la même signification que précédemment, composé de formule (IV) :

* <u>ou bien</u> que l'on déprotège par hydrolyse acide pour conduire au composé de formule (V) :

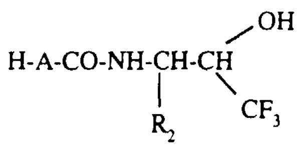 (V)

dans laquelle A et $R_2$ ont la même signification que précédemment,
sur lequel on fait réagir un amino-acide protégé de formule (VI), dont on a éventuellement séparé les isomères selon une technique classique de séparation, en présence d'un agent de couplage classique de la synthèse peptidique :

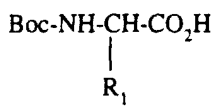 (VI)

dans laquelle Boc représente un radical butoxycarbonyle et $R_1$ a la même signification que dans la formule (I), pour conduire au composé de formule (VII) :

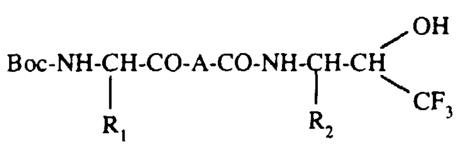 (VII)

dans laquelle Boc, $R_1$, A et $R_2$ ont la même signification que précédemment,
qui subit une oxydation pour conduire au composé de formule (VIII), dont on sépare éventuellement les isomères selon une technique classique de séparation,

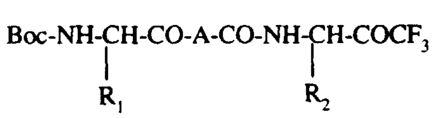 (VIII)

dans laquelle Boc, $R_1$, A et $R_2$ ont la même signification que précédemment,

* <u>ou bien</u> qui subit une oxydation pour conduire au composé de formule (X) :

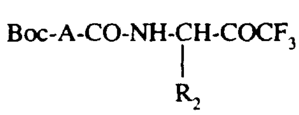 (X)

dans laquelle Boc, A et $R_2$ ont la même signification que précédemment,
que l'on déprotège en milieu acide pour conduire au composé de formule (XI) :

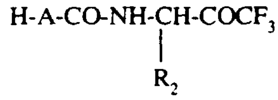

(XI)

dans laquelle A et $R_2$ ont la même signification que précédemment,
que l'on fait réagir sur un amino-acide protégé de formule (VI) tel que défini précédemment,
pour conduire au composé de formule (VIII) défini précédemment,

b soit sur un dipeptide protégé de formule (XII), obtenu par couplage classique de deux amino-acides sous forme racémique ou d'énantiomères purs,

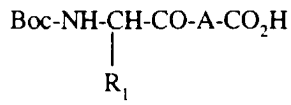

(XII)

dans laquelle Boc, $R_1$et A ont la même signification que précédemment,
pour conduire au composé de formule (VII) défini précédemment,
qui subit une oxydation et conduit au composé de formule (VIII) défini précédemment,

composé de formule (VIII) que l'on déprotège en milieu acide,
pour conduire au composé de formule (IX), dont on sépare éventuellement les isomères selon une technique classique de séparation,

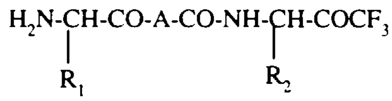

(IX)

dans laquelle $R_1$, A et $R_2$ ont la même signification que précédemment,
que l'on fait réagir sur un acide de formule (XIII), selon une technique classique de couplage peptidique,

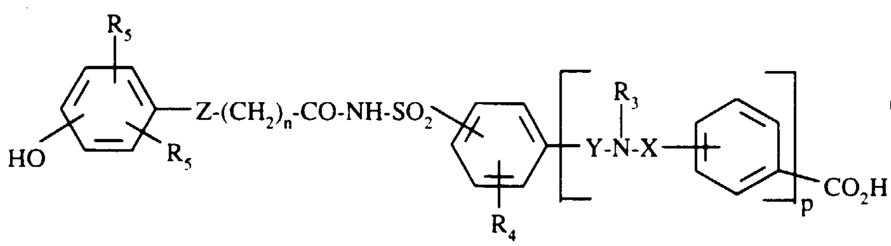

(XIII)

dans laquelle $R_5$, Z, n, p, $R_4$, $R_3$, X et Y ont la même signification que dans la formule (I), pour conduire au composé de formule (I),
composé de formule (I), que l'on purifie selon une technique classique de purification, dont on sépare, si on le désire, les isomères selon une technique classique de séparation, puis, si nécessaire, que l'on transforme en sel d'addition à une base pharmaceutiquement acceptable.

Les composés de formule (I) possèdent des propriétés pharmacologiques très intéressantes, en particulier inhibitrices de l'élastase leucocytaire humaine. A ce titre, ils peuvent être utilisés avec profit dans un certain nombre d'indications thérapeutiques comme l'emphysème pulmonaire, la bronchite chronique, le syndrome de détresse respiratoire aigue de l'adulte, la fibrose kystique, l'arthrite rhumatoïde, la glomérulonéphrite, les inflammations, les syndromes d'ischémie reperfusion, les phénomènes d'invasion et de diffusion des cellules malignes, les maladies dégénératives du tissu conjonctif, le vieillissement cutané.

L'activité inhibitrice de l'élastase leucocytaire humaine a été démontrée sur des tests in vitro et in vivo. Les composés ont présentés des activités inhibitrices supérieures aux produits de références tel que la chloromethylcétone ou la dichloroisocoumarine.

Les substituants des composés de formule (I) ont permis d'ajouter à l'activité inhibitrice d'élastase leucocytaire humaine, des propriétés de type anti-oxydant.

La présente invention a également pour objet les compositions pharmaceutiques renfermant comme principe actif au moins un composé de formule générale (I) ou un de ses sels d'addition à une base pharmacologiquement acceptable seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, les aérosols, les ampoules buvables et injectables...

La posologie utile varie selon l'âge et le poids du patient, la nature et la sévérité de l'affection ainsi que la voie d'administration.

Celle-ci peut être orale, nasale, rectale ou parentérale. D'une manière générale, la posologie unitaire s'échelonne entre 10 µg et 300 mg pour un traitement en 1 ou 3 prises par 24 heures.

Une voie préférentielle d'administration des dérivés de l'invention est la voie aérosol sous forme de poudre ou d'aérosol liquide.

Les exemples suivants illustrent l'invention et ne la limitent en aucune façon.

Les produits de départ utilisés sont des produits connus ou préparés selon des modes opératoires connus. Les abréviations utilisées dans les exemples sont les suivantes :

| | |
|---|---|
| Boc | à la place de tertbutoxycarbonyle, |
| Val | à la place de valyle, |
| Phi | à la place de perhydroindole-2-carbonyle, |
| Bz | à la place de benzyle, |
| Valinol-$CF_3$ | à la place de : |

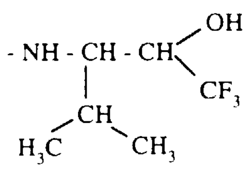

***Préparation A : Acide 4-[4-Chloro-3-(4-hydroxy-3,5-diterbutylphénylthioacétylaminosulfonyl)benzoylaminosulfonyl]benzoïque***

***Stade A : Acide 4-hydroxy-3,5-diterbutylphénylthioacétique***

144 mmoles de 4-hydroxy-3,5-diterbutylthiophénol, 128 mmoles de carbonate de potassium et 144 mmoles de bromoacétate de terbutyle sont agitées 7 heures, à 70°C, dans 280 ml d'acétonitrile, sous atmosphère d'azote, puis 12 heures à température ambiante. Après évaporation du solvant, le résidu est repris à l'eau et extrait par de l'éther éthylique. La phase organique est lavée par une solution saturée de chlorure de sodium, séchée et évaporée. Le résidu est purifié par chromatographie sur colonne de silice en utilisant comme éluant un mélange de chlorométhane/cyclohexane (40/60). L'huile recueillie est diluée dans 150 ml de chlorure de méthylène et 30 ml d'acide trifluoroacétique sont ajoutés à ce mélange. Après 18 heures à température ambiante, le solvant est évaporé et le produit attendu est obtenu après précipitation dans du pentane.
*Point de fusion : 130°C*

***Stade B: Ester éthylique de l'acide 4-chloro-3-(4-hydroxy-3,5-diterbutylphénylthioacétylaminosulfonyl)benzoïque***

A 89 mmoles du produit obtenu au stade précédent et 89 mmoles de 2-chloro-5-éthoxycarbonylbenzènesulfonamide dans 500 ml de tétrahydrofurane, sont ajoutées 17,9 mmoles de 2-diméthylaminopyridine et 93 mmoles de chlorhydrate de 1-(3-diméthylaminopropyl)-3-éthylcarbodiimide. L'ensemble est agité 20 heures à température ambiante. Après évaporation du solvant, le résidu est repris par de l'acétate d'éthyle. La phase organique est lavée par une solution d'acide citrique à 10 % puis par une solution saturée de chlorure de sodium. Après séchage et évaporation, le produit attendu est obtenu par cristallisation dans de l'éther isopropylique.
*Point de fusion : 144°C*

***Stade C: Acide 4-chloro-3-(4-hydroxy-3,5-diterbutylphénylthioacétylaminosulfonyl) benzoïque***

A 7,8 mmoles du composé obtenu au stade précédent dissoutes dans 300 ml d'éthanol sont ajoutées, sous atmosphère d'azote, 155 mmoles de soude 1N et 200 ml d'eau. Après 48 heures d'agitation, à température ambiante, l'éthanol est évaporé. La phase aqueuse résiduelle est lavée par de l'acétate d'éthyle puis acidifiée par de l'acide chlorhydrique. L'huile qui décante est extraite par de l'acétate d'éthyle. Après lavage de la phase organique par une solution saturée de chlorure de sodium, séchage et évaporation, le résidu est repris par du pentane et conduit au produit attendu qui cristallise.
*Point de fusion : 217°C*

***Stade D: Ester éthylique de l'acide 4-[-4-chloro-3-(4-hydroxy-3,5-diterbutylphénylthioacétylaminosulfonyl) benzoylaminosulfonyl]benzoïque***

Le produit attendu est obtenu par réaction de 58 mmoles du composé décrit au stade précédent avec 58 mmoles de 4-éthoxycarbonylbenzènesulfonamide selon le procédé décrit au stade B. Le produit attendu est purifié par chromatographie sur colonne de silice en utilisant comme éluant un mélange dichlorométhane/méthanol/ammoniaque à 28 % (90/10/0,5).
*Point de fusion : > 260°C*

***Stade E: Acide 4-[4-Chloro-3-(4-hydroxy-3,5-diterbutylphénylthioacétylaminosulfonyl)benzoylaminosulfonyl] benzoïque***

37 mmoles de l'ester obtenu au stade précédent sont saponifiées par 112 ml de soude 1N dans 250 ml d'éthanol et 150 ml d'eau selon la technique décrite au stade C.

***Exemple 1 : 4-[4-Chloro-3-(4-hydroxy-3,5-diterbutylphénylthioacétylaminosulfonyl)benzoylaminosulfonyl] benzoyl-(S)Val-(2S,3aS,7aS)Phi-(R,S)Val-$CF_3$***

***Stade A : Boc-(S)Val-(2S,3aS,7aS)Phi-OBz***

En utilisant la technique de couplage peptidique décrite par W. KONIG et R. GEIGER (Chem. Ber., 103, 788, 1970), on fait réagir 400 mmoles de Boc-(S)Val-OH et 400 mmoles de tosylate de (2S,3aS,7aS)Phi-OBz dans 700 ml de diméthylformamide (DMF). Après filtration de la dicyclohexylurée formée et évaporation du DMF, le résidu est dissous dans de l'acétate d'éthyle. La phase organique est lavée par de l'hydrogénocarbonate de sodium, de l'acide citrique à 10 % et par une solution saturée de chlorure de sodium. Après évaporation du solvant, le résidu est repris par de l'oxyde d'isopropyle. La solution est filtrée et traitée 1 heure à température ambiante par 5 g de Noir 50S. Le produit attendu est alors obtenu après filtration.

***Stade B: Boc-(S)Val-(2S,3aS,7aS)Phi-OH***

Le composé obtenu au stade précédent est dissous dans 500 ml d'éthanol, est hydrogéné pendant 20 heures, à température et pression ambiantes, en présence de 5 g de Palladium sur charbon à 5 %. Après filtration et évaporation, le résidu est repris au pentane, filtré et conduit au produit attendu.

***Stade C: Boc-(S)Val-(2S,3aS,7aS)Phi-(R,S)Valinol-$CF_3$***

20 mmoles du composé obtenu au stade précédent et 20 mmoles de chlorhydrate de 1,1,1-trifluoro-3-amino-4-méthyl-2-pentanol sont couplées selon la technique de couplage peptidique décrite au stade A. Le résidu final obtenu après lavage de la phase acétate d'éthyle et évaporation est repris par de l'éther. Un premier mélange de diastéréoisomères est filtré. L'éther est évaporé et le second mélange de diastéréosiomères est obtenu après purification du résidu par chromatographie sur colonne de silice en utilisant comme éluant un mélange dichlorométhane/acétate d'éthyle (90/10). Ces mélanges de diastéréoisomères sont utilisés tels quels au stade suivant.

***Stade D : Boc-(S)Val-(2S,3aS,7aS)Phi-(R,S) Val-CF3***

172 mmoles de chlorure d'oxalyle sont placées dans 50 ml de dichlorométhane, sous atmosphère d'azote. L'ensemble est refroidi à -55°C et 35 ml de diméthylsulfoxyde dans 100 ml de dichlorométhane sont ajoutés à cette température. Après 5 minutes d'agitation et en maintenant la température, 73 mmoles du composé obtenu au stade

précédent dans 300 ml de dichlorométhane sont ajoutées lentement. La température est alors amenée à -15°C et l'ensemble est maintenu 10 mn sous agitation. Après un nouveau refroidissement à -55,-60°C, 75 ml de triéthylamine dans 100 ml de dichlorométhane sont additionnés. Après retour à température ambiante, addition de 300 ml d'eau, la phase organique est décantée puis lavée par une solution d'acide citrique à 10 % jusqu'à pH acide puis par une solution de chlorure de sodium. Après séchage et évaporation, le résidu est purifié par chromatographie sur colonne de silice en utilisant comme éluant un mélange dichlorométhane/éthanol (97,5/2,5) et conduit au produit attendu.

***Stade E:(S)-Val-(2S,3aS,7aS)Phi-(R,S)Val-$CF_3$, chlorhydrate***

Le produit obtenu au stade précédent est dissous dans 350 ml de dioxane et un courant gazeux d'acide chlorhydrique est maintenu dans cette solution pendant 30 minutes à température ambiante. L'agitation est maintenue 15 heures. Le solvant est évaporé et on obtient le produit attendu qui est séché.

***Stade F : 4-[4-Chloro-3-(4-hydroxy-3,5-diterbutylphénylthioacétylaminosulfonyl)benzoylaminosulfonyl]benzoyl-(S)Val-(2S,3aS,7aS)Phi-(R,S)Val-$CF_3$***

Le produit attendu est obtenu selon le procédé décrit dans Tet. Lett., 1219, 1975 à partir du composé obtenu au stade précédent et de 30 mmoles de l'acide décrit dans la préparation A agitées dans 350 ml de DMF. Après 20 heures d'agitation, à température ambiante, le solvant est évaporé et le résidu repris par de l'acétate d'éthyle et une solution à 10 % d'acide citrique. La phase organique est décantée, lavée à l'eau jusqu'à neutralité et évaporée. Le résidu est purifié par chromatographie sur colonne de silice en utilisant comme éluant un mélange dichlorométhane/méthanol/ammoniaque à 28 % (85/15/0,5). Le produit attendu est ainsi obtenu et est lavé par une solution d'acide citrique à 10 % puis à l'eau jusqu'à neutralité. Après séchage et évaporation, il est repris au pentane et est filtré.

| *Microanalyse élémentaire :* | | | | | |
|---|---|---|---|---|---|
| | *C %* | *H %* | *N %* | *S %* | *Cl %* |
| *calculé* | *54,66* | *5,78* | *6,37* | *8,75* | *3,23* |
| *trouvé* | *54,82* | *5,93* | *6,69* | *8,69* | *3,48* |

***Exemple 2 : 4-[4-Chloro-3-(4hydroxy-3,5-diterbutylphénylthioacétylaminosulfonyl)benzoylaminosulfonyl]benzoyl-(S)Val-(2S,3aS,7aS)Phi-Val-$CF_3$, isomère*** $\alpha$

***Exemple 3 : 4-[4-Chloro-3-(4-hydroxy-3,5-diterbutylphénylthioacétylaminosulfonyl)benzoylaminosulfonyl]benzoyl-(S)Val-(2S,3aS,7aS)Phi-Val-$CF_3$, isomère*** $\beta$

Les isomères $\alpha$ et $\beta$ du composé de l'exemple 1 sous forme de sel de sodium sont séparés par chromatographie liquide sur colonne de silice C18 en utilisant comme éluant un mélange acétonitrile/hydrogénocarbonate de sodium (0,05M) selon un gradiant allant de 30 % à 100 % d'acétonitrile.

***Exemple 4 : [4-Chloro-3-(4-hydroxy-3,5-diterbutylphényl-thioacétylaminosulfonyl)benzoyl]-(S)Val-(2S,3aS,7aS)Phi-(R,S)Val-$CF_3$ [Composé non revendiqué]***

Le produit attendu est obtenu selon le procédé décrit dans l'exemple 1 en remplacant au stade F l'acide décrit dans la préparation A par l'acide décrit au stade C de la préparation A. Il est purifié par chromatographie sur colonne de silice en utilisant comme éluant un mélange dichlorométhane/méthanol/ammoniaque (90/10/1).

| *Microanalyse élémentaire :* | | | | | |
|---|---|---|---|---|---|
| | *C %* | *H %* | *N %* | *S %* | *Cl %* |
| *calculé* | *56,41* | *6,39* | *6,12* | *7,00* | *3,87* |
| *trouvé* | *56,58* | *6,81* | *6,07* | *6,80* | *3,96* |

## Etude pharmacologique des dérivés de l'invention

### *Exemple 5 : Activité inhibitrice de l'élastase leucocytaire humaine in vitro*

La puissance des composés de l'invention est déterminée par le niveau d'inhibition de l'action de l'élastase leucocytaire humaine sur un substrat peptidique de bas poids moléculaire selon la technique décrite par B.M. ASHE et coll. (J. Biol. Chem., 256, 11603-11606, 1981). Cette activité est mesurée en suivant la cinétique d'hydrolyse du substrat qui entraîne la libération de paranitroaniline qui absorbe à une longueur d'onde de 410 nm.

Réactif :

Enzyme : Elastase de sputum leucocytaire humain (Elastin Products Co.) solubilisée à 1000 UI/ml d'eau distillée, et congelée en aliquots de 50 µl à -20°C.
Substrat : méthoxysuccinyl-L-alanyl-L-alanyl-prolyl-valine-paranitroanilide (Sigma Chimie). Tampon : Tris 0,1 M ; NaCl 0,5 M ; pH = 7,8.

Matériel :

Spectrophotomètre thermostaté à 37°C équipé d'un passeur de cuve.
Cuve de 1 ml en polystyrène.
Bain-marie à sec réglé à 37°C.

Mode opératoire :

Dans une cuve on introduit :

- 970 µl de tampon
- 10 µl du produit à tester ou du solvant (concentré x 100)
- 10 µl d'élastase leucocytaire de sputum humain dilué au 1/10ème
- Agitation et incubation 15 mn à 37°C
- La réaction est débutée par ajout de 10 µl de substrat.

La cuve est introduite dans le spectrophotomètre, la densité optique est enregistrée en fonction du temps à 410 nm à 37°C. La vitesse initiale est mesurée pour chaque concentration de produit (ou témoin solvant) étudiée. Des pourcentages d'inhibition par rapport au témoin solvant sont calculées :

Pourcentage d'inhibition = 100 x [(vitesse témoin - vitesse du produit testé)/vitesse témoin].

Les concentrations inhibitrices 50 ($IC_{50}$) sont calculées à partir des pourcentages d'inhibition par régression linéaire simple. Les résultats obtenus dans ce test sont pour le composé de l'exemple 1 $IC_{50}$ = 26 nM, pour le composé de l'exemple 4: $IC_{50}$ = 46 nM.

### *Exemple 6 : Activité inhibitrice in vivo : modèle d'oedème aigu hémorragique chez le hamster*

L'instillation trachéale chez le hamster d'une préparation purifiée d'élastase leucocytaire de sputum humain entraîne une hémorragie aigue qui peut être quantifiée par la mesure de la concentration d'hémoglobine dans le lavage broncho-alvéolaire 18 heures après l'instillation d'élastase.
L'étude est effectuée chez les hamsters mâles de 120 à 140 g. (Syrian Golden, n = 10 par lot). Les animaux sont anesthésiés par du pentobarbital à la dose de 40 mg/kg par voie intrapéritonéale.
Les hamsters sont anesthésiés et la trachée est exposée chirurgicalement. Les produits à tester sont administrés à l'aide d'une aiguille directement dans la trachée dans un volume de 0,1 ml à la dose de 15 nM. L'élastase leucocytaire de sputum humain est administrée 18 heures après l'administration du produit par voie intrachéale, à la dose 50 unités par animal, sous un volume de 0,2 ml.
Les animaux sont sacrifiés à l'aide d'une dose léthale de pentobarbital, 3 heures après l'instillation de l'élastase, et un lavage broncho-alvéolaire au sérum physiologique est effectué. Le degré d'hémorragie est quantifié par méthode colorimétrique permettant le dosage de la concentration d'hémoglobine (Boeringer test-combination hémoglobine).

Les résultats sont exprimés en pourcentage d'inhibition de l'hémorragie.

Le composé de la présente invention est un inhibiteur effectif de l'élastase leucocytaire humaine qui prévient ou diminue l'hémorragie induite par l'instillation intratrachéale d'élastase leucocytaire humaine. Les résultats obtenus dans ce test pour le composé de l'exemple 1 démontrent une durée d'action remarquable avec une inhibition de 53 % au temps 18 h après administration intratrachéale et pour le composé de l'exemple 4, une inhibition de 23 % dans les mêmes conditions.

***Exemple 7 : Etude de la péroxydation lipidique***

A/ L'étude a été pratiquée sur microsomes hépatiques de rat en présence de $Fe^{3}$+ (100 μM) et d'ascorbate (100 μM). Le dosage du malondialdéhyde (MDA) est réalisé par la méthode à l'acide thiobarbiturique (spectrophotométrie λ = 532 nm) selon la technique décrite par N. PAYA et coll (Biochem. Pharmacol., vol. 44, n° 2, p. 205-214, 1992).

Le produit de l'exemple 1 possède une activité inhibitrice 50 % ($IC_{50}$) de $10^{-6}$M sur la péroxydation lipidique dans le système étudié.

B/ L'action des dérivés de la présente invention a été étudiée sur la modification oxydative des LDL induite par le sulfate de cuivre.

Les LDL humaines sont incubées 24 heures en présence de sulfate de cuivre $10^{-5}$M et en absence ou en présence des composés testés ($10^{-9}$M à $10^{-4}$M). Après incubation, la peroxydation des LDL est évaluée par électrophorèse sur gel d'agar et par la formation de malondialdéhyde (MDA) (Patharsrathy S et al. J. Clin. Invest. 77, 641-644, 1986).

L'activité des produits est évaluée par le calcul des concentrations réduisant de 50 % ($IC_{50}$) la production de MDA par rapport aux expériences contrôles.

Les composés des exemples 1 et 4 présentent une $IC_{50}$ de $3.10^{-7}$M sur la peroxydation des LDL humaines induite par le sulfate de cuivre.

***Exemple 8 : Composition pharmaceutique***

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

**Revendications**

1. Composés de formule (I) :

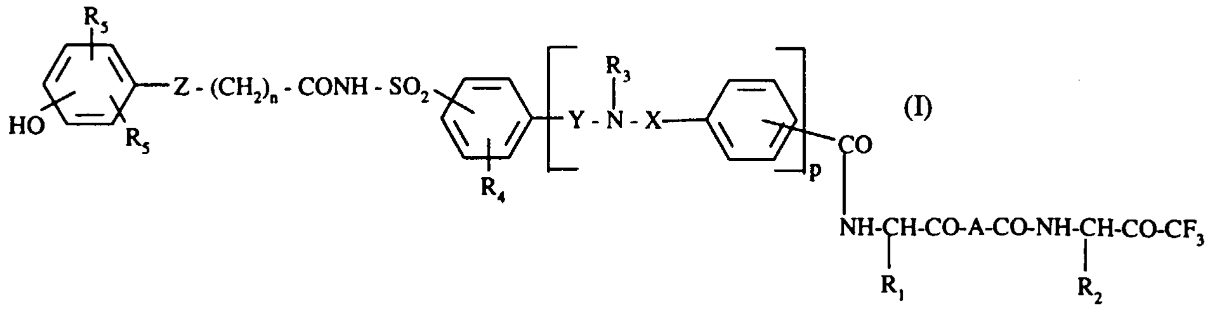

(I)

dans laquelle :

- $R_1$ représente un groupement alkyle ($C_1$-$C_6$) linéaire ou ramifié ou cycloalkyle ($C_3$-$C_7$),
- $R_2$ représente un groupement alkyle ($C_1$-$C_6$) linéaire ou ramifié ou cycloalkyle ($C_3$-$C_7$),
- $R_3$ représente un atome d'hydrogène ou un groupement alkyle ($C_1$-$C_6$) linéaire ou ramifié,
- $R_4$ représente un atome d'halogène ou un groupement alkyle ($C_1$-$C_6$) linéaire ou ramifié ou alkoxy ($C_1$-$C_6$) linéaire ou ramifié,
- $R_5$ représente un groupement alkyle ($C_1$-$C_6$) linéaire ou ramifié,
- X, Y, différents représentent CO ou $SO_2$,
- n représente 1, 2 ou 3,
- p représente 1
- Z représente un atome de soufre ou d'oxygène,
- A représente l'un quelconque des groupements suivants :

  •

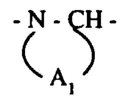

  dans lequel :

  $A_1$ représente avec les atomes d'azote et de carbone auquel il est attaché un cycle 2-azabicyclo[2.2.2]octane, 2-azabicyclo[2.2.1]heptane, perhydroindole, perhydroisoindole, indoline, isoindoline, perhydroquinoléine, perhydroisoquinoléine, 1,2,3,4-tétrahydroisoquinoléine, 1,2,3,4-tétrahydroquinoléine, cyclopenta[b]pyrolidine, 1,3-thiazolidine ou pyrrolidine,

  • ou,

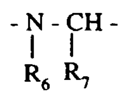 ,

  dans lequel :

  $R_6$ représente un atome d'hydrogène, un groupement alkyle (C1-C6) linéaire ou ramifié, un groupement cycloalkyle (C3-C6) ou un groupement indan-2-yle,
  $R_7$ représente un atome d'hydrogène ou un groupement alkyle (C1-C6) linéaire ou ramifié,

composés de formule (I) qui comprennent les hydrates de la fonction cétone $COCF_3$ correspondants,
leurs énantiomères, diastéréoisomères et épimères ainsi que leurs sels d'addition à une base pharmaceutiquement acceptable.

**2.** Composés de formule (I) selon la revendication 1 tels que A représente le groupement

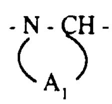

tel que défini dans la revendication 1.

**3.** Composés de formule (I) selon la revendication 1 tels que A représente un cycle perhydroindole.

**4.** Composés de formule (I) selon la revendication 1 tels que n est égal à 1.

**5.** Composés de formule (I) selon la revendication 1 tels que Z représente un atome de soufre.

**6.** Composé de formule (I) selon la revendication 1 qui est le 4-[4-chloro-3-(4-hydroxy-3,5-diterbutylphénylthioacétylaminosulfonyl)benzoylaminosulfonyl]benzoyl-(S)Val-(2S,3aS,7aS) Phi-(R,S)Val-$CF_3$

**7.** Procédé de préparation des composés de formule (I) selon la revendication 1 caractérisé en ce que l'on utilise, comme produit de départ un alcool de formule (II), dont on a éventuellement séparé les isomères par une technique classique de séparation :

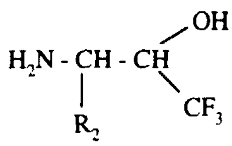

(II)

dans laquelle $R_2$ a la même signification que dans la formule (I),
que l'on fait réagir:

a soit sur un amino-acide protégé de formule (III), dont on a éventuellement séparé les isomères selon une technique classique de séparation, par une technique classique de couplage peptidique:

$$Boc\text{-}A\text{-}CO_2H \qquad (III)$$

dans laquelle A a la même signification que dans la formule (I) et Boc représente un groupement tertbutoxycarbonyle,
pour conduire au composé de formule (IV) :

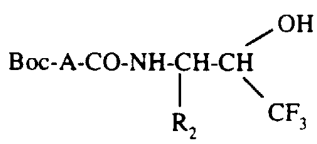

(IV)

dans laquelle A, $R_2$ et Boc ont la même signification que précédemment,
composé de formule (IV):

* ou bien que l'on déprotège par hydrolyse acide pour conduire au composé de formule (V) :

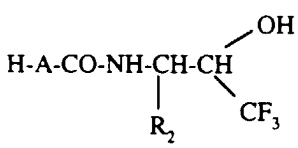

(V)

dans laquelle A et $R_2$ ont la même signification que précédemment,
sur lequel on fait réagir un amino-acide protégé de formule (VI), dont on a éventuellement séparé les isomères selon une technique classique de séparation, en présence d'un agent de couplage classique de la synthèse peptidique :

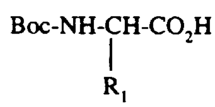

(VI)

dans laquelle Boc représente un radical butoxycarbonyle et $R_1$ a la même signification que dans la formule (I),
pour conduire au composé de formule (VII) :

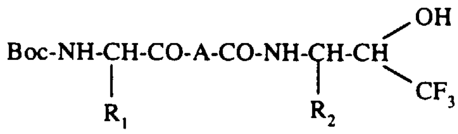 (VII)

dans laquelle Boc, $R_1$, A et $R_2$ ont la même signification que précédemment, qui subit une oxydation pour conduire au composé de formule (VIII), dont on sépare éventuellement les isomères selon une technique classique de séparation,

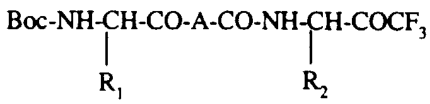 (VIII)

dans laquelle Boc, $R_1$, A et $R_2$ ont la même signification que précédemment,

* ou bien qui subit une oxydation pour conduire au composé de formule (X) :

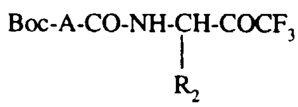 (X)

dans laquelle Boc, A et $R_2$ ont la même signification que précédemment, que l'on déprotège en milieu acide pour conduire au composé de formule (XI) :

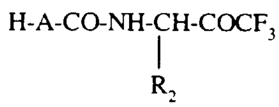 (XI)

dans laquelle A et $R_2$ ont la même signification que précédemment, que l'on fait réagir sur un amino-acide protégé de formule (VI) tel que défini précédemment, pour conduire au composé de formule (VIII) défini précédemment,

b soit sur un dipeptide protégé de formule (XII), obtenu par couplage classique de deux amino-acides sous forme racémique ou d'énantiomères purs,

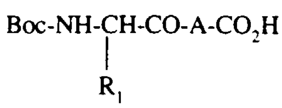 (XII)

dans laquelle Boc, $R_1$ et A ont la même signification que précédemment, pour conduire au composé de formule (VII) défini précédemment, qui subit une oxydation et conduit au composé de formule (VIII) défini précédemment,

composé de formule (VIII) que l'on déprotège en milieu acide, pour conduire au composé de formule (IX), dont on sépare éventuellement les isomères selon une technique classique de séparation,

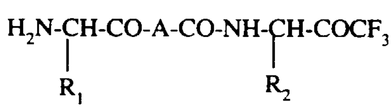 (IX)

dans laquelle $R_1$, A et $R_2$ ont la même signification que précédemment,

que l'on fait réagir sur un acide de formule (XIII), selon une technique classique de couplage peptidique,

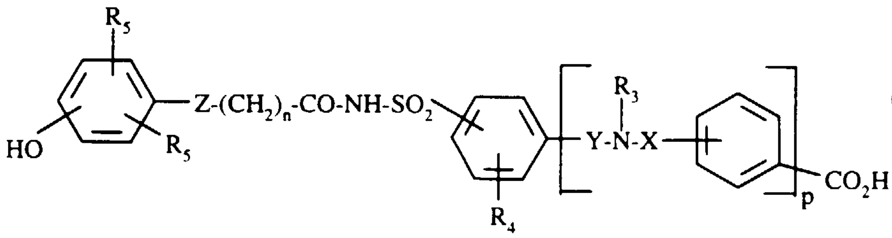

(XIII)

dans laquelle $R_5$, Z, n, p, $R_4$, $R_3$, X et Y ont la même signification que dans la formule (I),
pour conduire au composé de formule (I),
composé de formule (I), que l'on purifie selon une technique classique de purification, dont on sépare, si on le désire, les isomères selon une technique classique de séparation, puis, si nécessaire, que l'on transforme en sel d'addition à une base pharmaceutiquement acceptable.

**8.** Compositions pharmaceutiques contenant comme principe actif au moins un composé selon l'une quelconque des revendications 1 à 6 seul ou en combinaison avec un ou plusieurs excipients ou véhicules inertes, non toxiques, pharmaceutiquement acceptables.

**9.** Compositions pharmaceutiques selon la revendication 9 contenant au moins un principe actif selon l'une quelconque des revendications 1 à 6 utilisé en tant qu'inhibiteur de l'élastase leucocytaire humaine à titre de traitement principal ou complémentaire des maladies dégénératives du tissu conjonctif comme l'emphysème pulmonaire, des troubles inflammatoires comme l'arthrite rhumatoïde, des pathologies pulmonaires du type du syndrome de détresse respiratoire aigu, de la fibrose kystique, de la bronchite chronique, des lésions tissulaires induites par le syndrome d'ischémie reperfusion et en tant que traitement topique sur la peau afin d'inhiber l'élastolyse.

## Claims

**1.** Compounds of formula (I):

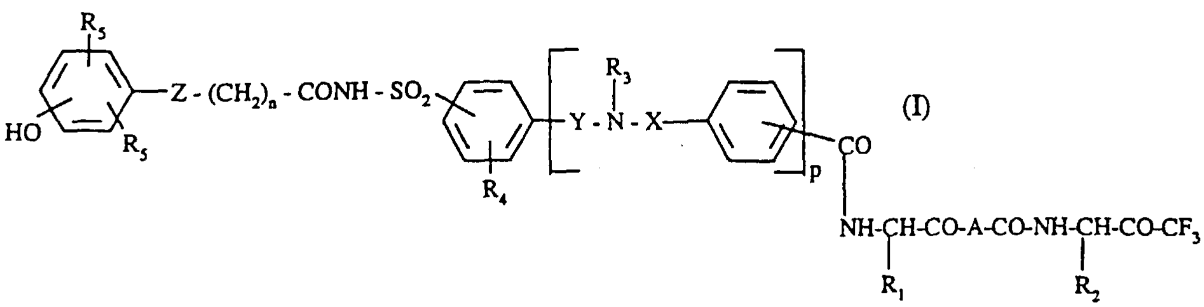

(I)

in which:

$R_1$ represents a linear or branched ($C_1$-$C_6$)alkyl group, or a ($C_3$-$C_7$)cycloalkyl group,
$R_2$ represents a linear or branched ($C_1$-$C_6$)alkyl group, or a ($C_3$-$C_7$)cycloalkyl group,
$R_3$ represents a hydrogen atom or a linear or branched ($C_1$-$C_6$)alkyl group,
$R_4$ represents a halogen atom or a linear or branched ($C_1$-$C_6$)alkyl group or a linear or branched ($C_1$-$C_6$)alkoxy group,
$R_5$ represents a linear or branched ($C_1$-$C_6$)alkyl group,
X and Y are different and represent CO or $SO_2$,
n represents 1, 2 or 3,
p represents 1,
Z represents a sulphur atom or an oxygen atom, and
A represents any one of the following groups:

- 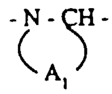

  in which:
  $A_1$ represents, with the nitrogen and carbon atoms to which it is bonded, a 2-azabicyclo[2.2.2]octane, 2-azabicyclo[2.2.1]heptane, perhydroindole, perhydroisoindole, indoline, isoindoline, perhydroquinoline, perhydroisoquinoline, 1,2,3,4-tetrahydroisoquinoline, 1,2,3,4-tetrahydroquinoline, cyclopenta[b]pyrrolidine, 1,3-thiazolidine or pyrrolidine ring,
- or

  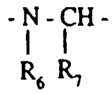 ,

  in which:

  $R_6$ represents a hydrogen atom, a linear or branched ($C_1$-$C_6$)alkyl group, a ($C_3$-$C_6$)cycloalkyl group or an indan-2-yl group,
  $R_7$ represents a hydrogen atom or a linear or branched ($C_1$-$C_6$)alkyl group,

which compounds of formula (I) include the corresponding hydrates of the $COCF_3$ ketone function,
their enantiomers, diastereoisomers and epimers, and addition salts thereof with a pharmaceutically acceptable base.

**2.** Compounds of formula (I) according to claim 1 wherein A represents the group

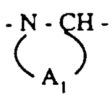

as defined in claim 1.

**3.** Compounds of formula (I) according to claim 1 wherein A represents a perhydroindole ring.

**4.** Compounds of formula (I) according to claim 1 wherein n is equal to 1.

**5.** Compounds of formula (I) according to claim 1 wherein Z represents a sulphur atom.

**6.** Compound of formula (I) according to claim 1 which is 4-[4-chloro-3-(4-hydroxy-3,5-di-tert-butylphenylthioacetylaminosulphonyl)benzoylaminosulphonyl]benzoyl(S)Val-(2S,3aS,7aS)-Phi-(R,S)Val-$CF_3$.

**7.** Process for the preparation of compounds of formula (I) according to claim 1, characterised in that there is used as starting material an alcohol of formula (II), which has optionally been separated into its isomers by a conventional separation technique:

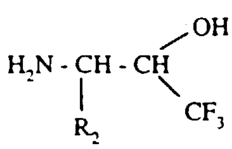 (II),

in which $R_2$ is as defined in formula (I),
which is reacted:

a either with a protected amino acid of formula (III), which has optionally been separated into its isomers by a conventional separation technique, by a conventional peptide coupling technique:

$$\text{Boc - A - CO}_2\text{H} \quad \text{(III)},$$

in which A is as defined in formula (I) and Boc represents a tert-butoxycarbonyl group,
to yield a compound of formula (IV):

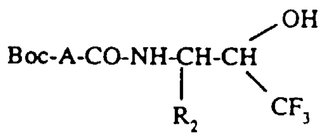
(IV),

in which A, $R_2$ and Boc are as defined above,
which compound of formula (IV):

* either is deprotected by acid hydrolysis to yield a compound of formula (V):

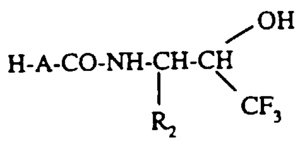
(V),

in which A and $R_2$ are as defined above,
which is reacted with a protected amino acid of formula (VI), which has optionally been separated into its isomers by a conventional separation technique, in the presence of a conventional coupling agent for peptide synthesis:

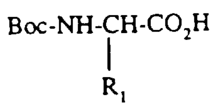
(VI),

in which Boc represents a butoxycarbonyl radical and $R_1$ is as defined in formula (I),
to yield a compound of formula (VII):

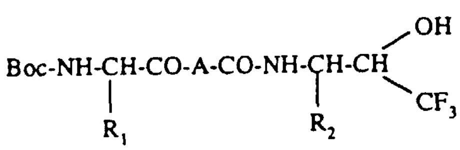
(VII),

in which Boc, $R_1$, A and $R_2$ are as defined above,
which is subjected to oxidation to yield a compound of formula (VIII), which is optionally separated into its isomers by a conventional separation technique,

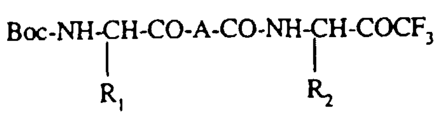
(VIII),

in which Boc, $R_1$, A and $R_2$ are as defined above,

* or is subjected to oxidation to yield a compound of formula (X):

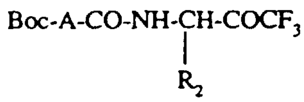

(X),

in which Boc, A and $R_2$ are as defined above,
which is deprotected in an acid medium to yield a compound of formula (XI):

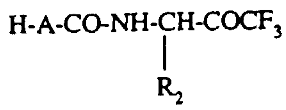

(XI),

in which A and $R_2$ are as defined above,
which is reacted with a protected amino acid of formula (VI) as defined above to yield a compound of formula (VIII) defined above,

b or with a protected dipeptide of formula (XII), obtained by conventional coupling of two amino acids in racemic form or in the form of pure enantiomers,

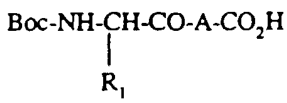

(XII),

in which Boc, $R_1$ and A are as defined above,
to yield a compound of formula (VII) defined above,
which is subjected to oxidation and yields a compound of formula (VIII) defined above,
which compound of formula (VIII) is deprotected in an acid medium to yield a compound of formula (IX), which is optionally separated into its isomers by a conventional separation technique,

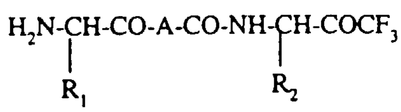

(IX),

in which $R_1$, A and $R_2$ are as defined above,
which is reacted with an acid of formula (XIII) according to a conventional peptide coupling technique,

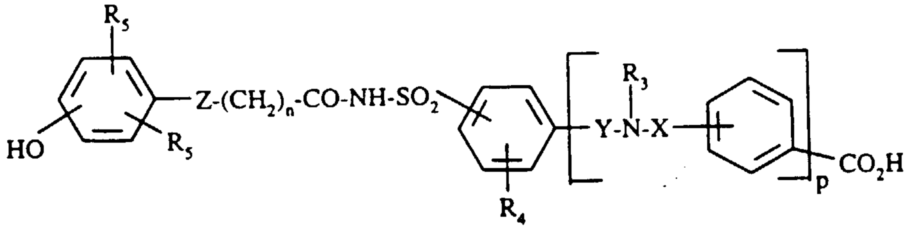

(XIII),

in which $R_5$, Z, n, p, $R_4$, $R_3$, X and Y are as defined in formula (I),
to yield a compound of formula (I),
which compound of formula (I) is purified by a conventional purification technique, is separated, if desired, into

its isomers by a conventional separation technique and then, if necessary, is converted into an addition salt with a pharmaceutically acceptable base.

**8.** Pharmaceutical compositions comprising as active ingredient at least one compound according to any one of claims 1 to 6, alone or in combination with one or more inert, non-toxic, pharmaceutically acceptable excipients or carriers.

**9.** Pharmaceutical compositions according to claim 8 comprising at least one active ingredient according to any one of claims 1 to 6, for use as an inhibitor of human leukocyte elastase as principal or complementary treatment for degenerative diseases of connective tissue, such as pulmonary emphysema, inflammatory disorders, such as rheumatoid arthritis, pulmonary pathologies of the acute respiratory distress syndrome type, cystic fibrosis, chronic bronchitis, tissue lesions induced by the syndrome of reperfusion ischaemia, and as a topical treatment on the skin in order to inhibit elastolysis.

## Patentansprüche

**1.** Verbindungen der Formel (I):

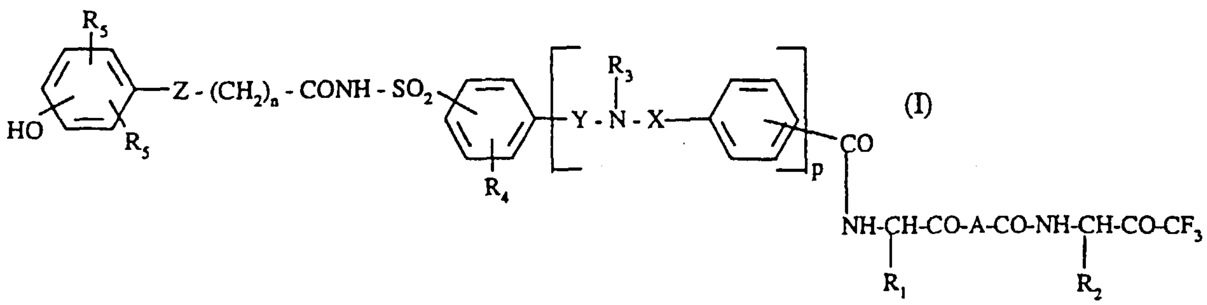

(I)

in der:

| | |
|---|---|
| $R_1$ | eine geradkettige oder verzweigte ($C_1$-$C_6$)-Alkylgruppe oder ($C_3$-$C_7$)-Cycloalkylgruppe, |
| $R_2$ | eine geradkettige oder verzweigte ($C_1$-$C_6$)-Alkylgruppe oder ($C_3$-$C_7$)-Cycloalkylgruppe, |
| $R_3$ | ein Wasserstoffatom oder eine geradkettige oder verzweigte ($C_1$-$C_6$)-Alkylgruppe, |
| $R_4$ | ein Halogenatom oder eine geradkettige oder verzweigte ($C_1$-$C_6$)-Alkylgruppe oder eine geradkettige oder verzweigte ($C_1$-$C_6$)-Alkoxygruppe, |
| $R_5$ | eine geradkettige oder verzweigte ($C_1$-$C_6$)-Alkylgruppe, |
| X und Y, | die verschieden sind, CO oder $SO_2$, |
| n | 1, 2 oder 3, |
| p | 1, |
| Z | ein Sauerstoffe- oder Schwefelatom und |
| A | eine der folgenden Gruppen: |

- 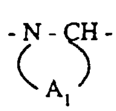 ,

  in der:

  $A_1$ zusammen mit dem Stickstoff- und dem Kohlenstoffatom, an das sie gebunden ist, einen 2-Azabicyclo[2.2.2]octan-, 2-Azabicyclo[2.2.1]heptan-, Perhydroindol-, Perhydroisoindol-, Indolin-, Isoindolin-, Perhydrochinolin-, Perhydroisochinolin-, 1,2,3,4-Tetrahydroisochinolin-, 1,2,3,4-Tetrahydrochinolin-, Cyclopenta[b]pyrrolidin-, 1,3-Thiazolidin- oder Pyrrolidin-Ring darstellt,

- oder

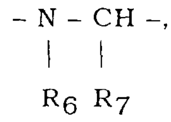

in der:

$R_6$ ein Wasserstoffatom, eine geradkettige oder verzweigte ($C_1$-$C_6$)-Alkylgruppe, eine ($C_3$-$C_6$)-Cycloalkylgruppe oder eine Indan-2-yl-gruppe und
$R_7$ ein Wasserstoffatom oder eine geradkettige oder verzweigte ($C_1$-$C_6$)-Alkylgruppe darstellen,

bedeuten,
Verbindungen der Formel (I), welche die entsprechenden Hydrate der Ketonfunktion $COCF_3$ umfassen,
deren Enantiomere, Diastereoisomere und Epimere sowie deren Additionssalze mit einer pharmazeutisch annehmbaren Base.

**2.** Verbindungen der Formel (I) nach Anspruch 1, worin A die Gruppe

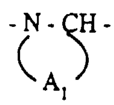

darstellt, wie sie in Anspruch 1 definiert ist.

**3.** Verbindungen der Formel (I) nach Anspruch 1, worin A einen Perhydroindolring bedeutet.

**4.** Verbindungen der Formel (I) nach Anspruch 1, worin n den Wert 1 besitzt.

**5.** Verbindungen der Formel (I) nach Anspruch 1, worin Z ein Schwefelatom bedeutet.

**6.** Verbindung der Formel (I) nach Anspruch 1, nämlich 4-[4Chlor-3-(4-hydroxy-3,5-di-tert.-butylphenylthioacetylaminosulfonyl)-benzoylaminosulfonyl]-benzoyl(S)Val-(2S,3aS,7aS)Phi-(R,S)-Val-$CF_3$.

**7.** Verfahren zur Herstellung der Verbindungen der Formel (I) nach Anspruch 1, dadurch gekennzeichnet, daß man als Ausgangsprodukt einen Alkohol der Formel (II):

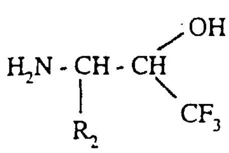

(II)

in der $R_2$ die bezüglich der Formel (I) angegebenen Bedeutungen besitzt,
den man gegebenenfalls mit Hilfe einer klassischen Trennmethode in die Isomeren aufgetrennt hat,

a entweder mit einer geschützten Aminosäure der Formel (III):

$$Boc - A - CO_2H \quad (III)$$

in der A die bezüglich der Formel (I) angegebenen Bedeutungen besitzt und Boc eine tert.-Butoxycarbonylgruppe darstellt, die man gegebenenfalls mit Hilfe einer klassischen Trennmethode in die Isomeren aufgetrennt hat, mit Hilfe einer klassischen Peptidkupplungstechnik umsetzt
zur Bildung der Verbindung der Formel (IV):

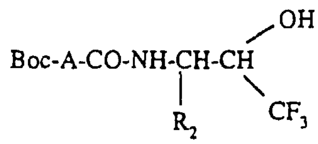

(IV)

in der A, $R_2$ und Boc die oben angegebenen Bedeutungen besitzen,
welche Verbindung der Formel (IV):

* man entweder einer Behandlung zur Abspaltung der Schutzgruppe durch saure Hydrolyse unterwirft zur Bildung der Verbindung der Formel (V):

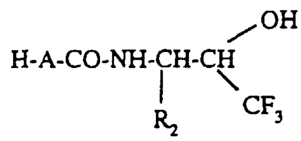

(V)

in der A und $R_2$ die oben angegebenen Bedeutungen besitzen,
welche man mit einer geschützten Aminosäure der Formel (VI):

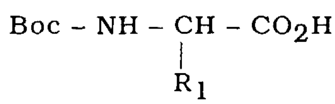

(VI)

in der Boc eine tert.-Butoxycarbonylgruppe bedeutet und $R_1$ die bezüglich der Formel (I) angegebenen Bedeutungen besitzen,
die man gegebenenfalls mit Hilfe einer klassischen Trennmethode in die Isomeren getrennt hat, in Gegenwart eines klassischen Kupplungsmittels für die Peptidsynthese umsetzt zur Bildung der Verbindung der Formel (VII):

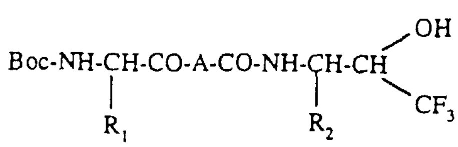

(VII)

in der Boc, $R_1$, A und $R_2$ die oben angegebenen Bedeutungen besitzen,
welche man einer Oxidation unterwirft zur Bildung der Verbindung der Formel (VIII):

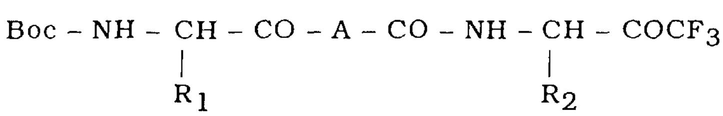

(VIII)

in der Boc, $R_1$, A und $R_2$ die oben angegebenen Bedeutungen besitzen, welche man gegebenenfalls mit Hilfe einer klassischen Trennmethode in die Isomeren auftrennt,

* oder man einer Oxidation unterwirft zur Bildung der Verbindung der Formel (X):

$$Boc - A - CO - NH - \underset{R_2}{\underset{|}{CH}} - COCF_3 \qquad (X)$$

in der Boc, A, und $R_2$ die oben angegebenen Bedeutungen besitzen,
von welcher man in saurem Medium die Schutzgruppe abspaltet zur Bildung der Verbindung der Formel (XI):

$$H - A - CO - NH - \underset{R_2}{\underset{|}{CH}} - COCF_3 \qquad (XI)$$

in der A und $R_2$ die oben angegebenen Bedeutungen besitzen,
welche man mit einer geschützten Aminosäure der Formel (VI), wie sie oben definiert worden ist, umsetzt, zur Bildung der Verbindung der oben definierten Formel (VIII),

b oder mit einem geschützten Dipeptid der Formel (XII):

$$Boc - NH - \underset{R_1}{\underset{|}{CH}} - CO - A - CO_2H \qquad (XII)$$

in der Boc, $R_1$ und A die oben angegebenen Bedeutungen besitzen,
welche man durch klassische Kupplung von zwei Aminosäuren in Form des Racemats oder der reinen Enantiomeren erhalten hat,
zur Bildung der Verbindung der oben definierten Formel (VII),
welche einer Oxidation unterworfen wird und zu der Verbindung der oben definierten Formel (VIII) führt,

von welcher Verbindung der Formel (VIII) man die Schutzgruppe in saurem Medium abspaltet,
zur Bildung der Verbindung der Formel (IX):

$$H_2N - \underset{R_1}{\underset{|}{CH}} - CO - A - CO - NH - \underset{R_2}{\underset{|}{CH}} - COCF_3 \qquad (IX)$$

in der $R_1$, A und $R_2$ die oben angegebenen Bedeutungen besitzen,
welche man gegebenenfalls mit Hilfe einer klassischen Trennmethode in die Isomeren auftrennt, welche man mit Hilfe einer klassischen Peptidkupplungstechnik mit einer Säure der Formel (XIII) umsetzt:

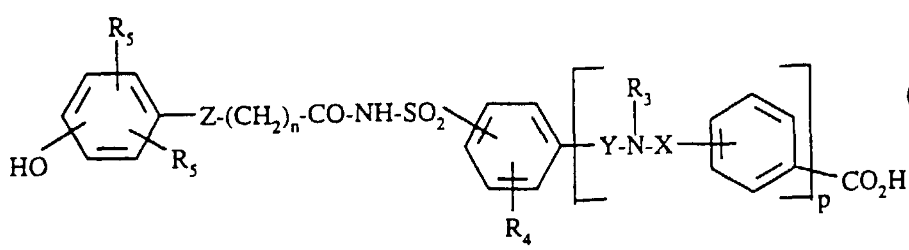

(XIII)

in der $R_5$, Z, n, p, $R_4$, $R_3$, X und Y die bezüglich der Formel (I) angegebenen Bedeutungen besitzen,

zur Bildung der Verbindung der Formel (I),
welche Verbindung der Formel (I) man mit Hilfe einer klassischen Reinigungsmethode reinigt, gewünschtenfalls mit Hilfe einer klassischen Trennmethode in die Isomeren auftrennt und erforderlichenfalls mit Hilfe einer pharmazeutisch annehmbaren Base in die Salze überführt.

**8.** Pharmazeutische Zubereitungen enthaltend als Wirkstoff mindestens eine Verbindung nach einem der Ansprüche 1 bis 6 allein oder in Kombination mit einem oder mehreren inerten, nichttoxischen, pharmazeutisch annehmbaren Trägermaterialien oder Bindemitteln.

**9.** Pharmazeutische Zubereitungen nach Anspruch 8, enthaltend mindestens einen Wirkstoff nach einem der Ansprüche 1 bis 6 zur Verwendung als Inhibitoren der menschlichem Leukozytenelastase bei der hauptsächlichen oder ergänzenden Behandlung von degenerativen Erkrankungen des Bindegewebes, wie Lungenemphysem, Entzündungszuständen, wie rheumatoide Arthritis, pathologischen Zuständen der Lungen des Typs des akuten Atemnotsyndroms, der kystischen Fibrose, der chronischen Bronchitis, von Gewebeverletzungen, die durch das Syndrom der Reperfusionsischämie ausgelöst worden sind, und zur topischen Behandlung der Haut zur Inhibierung der Elastolyse.